# EUROPÄISCHE PATENTSCHRIFT

(11) **EP 0 584 594 B1**
(45) Veröffentlichungstag und Bekanntmachung des Hinweises auf die Patenterteilung: **01.04.1998**
(21) Anmeldenummer: 93112471.3
(22) Anmeldetag: 04.08.1993
(51) Int. Cl.: A61K 9/00, A61K 9/20, A61K 9/28

(54) **Leicht einnehmbare brausende Manteltablette**
Easily taken effervescent manteltablets
Comprimés effervescents facile à administrer

(30) Priorität: 06.08.1992 CH 2468/92
(43) Veröffentlichungstag der Anmeldung: 02.03.1994
(73) Patentinhaber: Gergely, Gerhard, Dr., A-1050 Wien (AT)
(72) Erfinder: Gergely, Gerhard Dr., A-1050 Wien (AT); Tritthart, Wolfram Dr., A-9400 Wolfsberg (AT); Wolf, Rüdiger Dr., A-1080 Wien (AT)
(74) Vertreter: Büchel, Kurt F., Dr.

(56) Entgegenhaltungen:
- EP-A- 0 257 310
- BE-A- 666 198
- FR-A- 2 350 102
- US-A- 4 503 031
- US-A- 4 678 661
- D5: Hagers Handbuch der pharmazeutischen Praxis, seiten 688-689

## Beschreibung

Die Erfindung betrifft zur oralen Applikation bestimmte pharmazeutische Zubereitungen, die Wirkstoffe nach einem vorgegebenen Muster freisetzen.

Derartige Abgabesysteme sind unter anderem in der DE-A1-3821426 beschrieben. Auch Mehrschicht-Tabletten und Manteltabletten erfüllen dieses Erfordernis, vgl. DE-A1-3506895, GB-A-800973 und GB-A-2056278. Der Mantel kann bei derartigen Tabletten auch aus Brausematerialien bestehen, so daß die in der Mantelschicht inkorporierten Substanzen und Wirkstoffe rasch in die umgebende Flüssigkeit freigesetzt werden, z.B. wie in der US-A-536155. Insbesondere sogenannte Magen-Darm-Pillen bestehen oftmals aus einem mit einem magensaftresistenten Film umgebenen Heilmittelkern und einem im Mund oder im Magensaft leicht löslichen Mantel, vgl. CH-A-649216 und DE-A1-3403329.

Eine Manteltablette besteht namensgemäß aus einem Kern und einem Mantel. Dies ermöglicht einen Schutz der im Kern verarbeiteten Wirkstoffe vor Feuchtigkeit und Lichteinwirkung; eine Kaschierung unangenehmer organoleptischer Eigenschaften von wirkstoffen durch deren Einarbeitung im Kern; eine Trennung von inkompatiblen Wirkstoffen innerhalb der Tablette; eine Steuerung der Wirkstofffreisetzung durch unterschiedliche Freigaberaten aus Kern und Mantel; eine Steuerung der Resorption im Gastrointestinaltrakt durch verschiedene Löslichkeiten von Mantel und Kern in Abhängigkeit von der Wasserstoffionenkonzentration bzw. der enzymatischen Umgebung.

Auch können Manteltabletten, wie normale Tabletten, magensaftresistente Überzüge besitzen oder dragiert werden, so daß eine pharmakokinetisch günstige Steuerung der Wirkstofffreigabe und Resorption erzielt werden kann. Dabei können auch bereits überzogene Kerne nochmals ummantelt werden.

Zahlreiche Patienten haben jedoch mit dem Schlucken ungeteilter fester Arzneiformen Schwierigkeiten. Die Einnahme nicht-zerkleinerter Arzneiformen bereitet vielen solche Ekelgefühle, daß dragierte und insbesondere Manteltabletten von ihnen offen oder versteckt abgelehnt werden. Es ist daher oftmals erforderlich, daß der Arzt, eine Schwester oder eine Bezugsperson - bei Kindern zumeist die Mutter oder der Vater - zur Sicherung der Therapie die unmittelbare Einnahme der Tablette kontrolliert. Tabletten können aber leicht im Mund versteckt und später wieder ausgespuckt werden. Eine echte Kontrolle der Tabletteneinnahme ist insbesondere bei Erwachsenen daher kaum möglich. Die Arzneimitteldosis sollte ferner aus psychologischen Gründen möglichst in einer einzigen Tablette verabreicht werden, insbesondere wenn die Wirkstoffeinnahme regelmäßig erfolgen soll. Höhere Dosen bzw. größere Substanzmengen lassen sich aber oftmals nur schwer in einer einzigen Tablette unterbringen.

Die DE-A1-39 00 811 lehrt, bei Wirkstoffen mit unangenehmen organoleptischen Eigenschaften diese in Partikel mit einer in Wasser im wesentlichen unlöslichen, aber im Magensaft innerhalb 15 Minuten sich lösenden Hülle einzuschließen und die so umhüllten Partikel zu einem in Wasser suspendierbaren, pulver- oder granulatartigen Gemenge zu formulieren. Die Dichte und Größe der Partikel sind so gewählt, daß die Zubereitung sich leicht in Wasser zu einer weitgehend stabilen Suspension suspendieren läßt. Die einzelnen Partikel besitzen einen Durchmesser von höchstens ca. 1,5 mm. Bei einer derartigen pharmazeutischen Zubereitung ist jedoch eine exakte Dosierung schwierig. Das pulver- oder granulatartige Gemenge muß mit einem Löffel, nach Gewicht oder sonstwie abgemessen werden. Wird dies von Patienten oder Laien vorgenommen, so führt dies regelmäßig zu Über- oder Unterdosierungen. Auch sind nicht alle Vorteile der Manteltabletten gegeben: insbesondere ist eine Steuerung der Wirkstofffreisetzung durch unterschiedliche Freigaberaten aus Kern und Mantel nicht möglich - die Wirkstoffe werden vielmehr einheitlich im Magen oder Gastrointestinaltrakt freigesetzt; auch können inkompatible Wirkstoffe nicht zu einer einheitlichen Formulierung verarbeitet werden, da die Bestandteile in den Pellets nicht getrennt vorliegen.

In US-A-4503031 wird eine zweilagige Doppelfunktionstablette zur direkten oralen Anwendung beschrieben. Der kleinere, mit geringer Menge Brause versetzte Anteil der Tablette ermöglicht die sofortige Freisetzung des darin enthaltenen Wirkstoffs nach der Einnahme, während der zweite, grössere Anteil aus unterschiedlich dick gekapselten Wirkstoffpellets besteht, und so eine gleichmässige Abgabe über einen längeren Zeitraum bewirkt. Es werden dabei keine Vorkehrungen zu einer leichteren Einnahme oder zur gleichzeitigen Applikation mehrerer Wirkstoffe getroffen.

EP-A-257310 zeigt, wie kleine Wirkstoffpartikel (ca. 400µm) mit einer dünnen flexiblen Schicht umhüllt werden, die ein Verpressen der Kapseln zu Tabletten erlaubt. Die Tablette zerfällt entweder im Magendarmtrakt oder in wässriger Lösung vollständig zu Mikropellets, die den Wirkstoff verzögert über ein grösseres Volumen des Magendarmtrakts freisetzen, als dies durch eine kompakte Arzneiform möglich wäre. Auch hier werden keine Vorkehrungen zur gleichzeitigen Applikation mehrerer Wirkstoffe getroffen. Weiters kann mit einer solchen Tablette kein beschleunigtes Anheben auf ein therapeutisches Niveau erfolgen, da der gesamte Wirkstoffanteil gekapselt ist.

Der Erfindung liegt daher die Aufgabe zugrunde, eine pharmazeutische Zubereitung zur Verfügung zu stellen, bei der die orale Einnahme trotz Tablettenform erleichtert ist, so daß alle Vorteile der Manteltabletten, insbesondere die Freisetzung der Wirkstoffe nach einem zeitlichen Muster, zur Verfügung stehen.

Diese Aufgabe wird durch eine oral einzunehmende pharmazeutische Formulierung gemäß Anspruch 1 gelöst. Insbesondere durch eine Mantellöstablette, bestehend aus einer oder mehreren, um einen Kern angeordneten, wirkstoffhaltigen, mantelbildenden, eine Brausemischung enthaltenden Schichten und einem vorzugsweise in eine Hülle eingeschlossenen Tablettenkern mit wenigstens einer Retardarzneiform, wobei die Manteldicke, die Lösgeschwindigkeit und die Brauseaktivität der Mantelzusammensetzungen so gewählt sind, daß in 100 ml Wasser sich der Mantel einer Tablette zwischen 30 und 180 Sekunden, bevorzugt zwischen 60 und 90 Sekunden, vollständig löst oder dispergiert. Bevorzugte Ausführungsformen der Erfindung ergeben sich aus den Unteransprüchen.

Zur oralen Applikation wird die neue pharmazeutische Formulierung zunächst in ein Gefäß mit Wasser gegeben, wobei sich die Mantelschichten rasch weglösen. Der Tablettenkern mit der Wirksubstanz in retardierter Form bleibt am Boden des Gefäßes liegen. Dies erfordert kein eigenes Dosieren oder Abmessen der Substanzen, denn die Dosen sind bei einer festen Tablette vorgegeben. Der Patient trinkt zunächst die wäßrige Lösung der Mantelschicht; die innere Tablette - der Tablettenkern - wird dann mit dem letzten Schluck der Lösung eingenommen und geschluckt.

Eine derartige Aufnahme unzerteilter Arzneimittel bereitet den meisten Patienten keinerlei Schwierigkeiten und erleichtert allgemein die Einnahme ungeteilter Arzneiformen. Zum einen werden durch das Trinken einer großen Menge Wasser vorab eventuelle Würgreize vermindert, zum anderen wird der Kern der Tablette schlicht weggetrunken. Die Einnahme der Tablette ist leicht zu kontrollieren, da beim Trinken schwer zugleich eine Tablette im Mund versteckt werden kann. Auch ist der Eindruck einer Tablettenaufnahme vermindert, da das gleichzeitige Schlucken von festen und flüssigen Stoffen einer normalen Nahrungsaufnahme weitgehend entspricht. Dies um so mehr, als der Brausemantel wasserlösliche Geschmacks-und Aromastoffe, gegebenenfalls auch Farbstoffe, Mineralstoffe, Vitamine, etc. enthalten kann, so daß nach Lösen des Tablettenmantels ein gefälliges und wohlschmeckendes Brausegetränk entsteht.

Um ein derartiges Brausegetränk zu erhalten, liegt das Gewicht des Brausemantels zwischen 1 und 15 g, bevorzugt zwischen 2 und 10 g, also im Bereich von 1 bis 3 Gew.% der für das Lösen der Tablette empfohlenen Wassermenge, die bei der Aufnahme von Tabletten typisch zwischen 100 und 200 ml liegt.

Erfindungsgemäße Mantelmaterialien enthalten zur Erhöhung der Lösgeschwindigkeit Brausemischungen aus Säurekomponenten und Bicarbonaten oder Carbonaten, vorzugsweise als lösliche Calciumsalze. Ferner kann der Tablettenmantel quellfähige Substanzen enthalten, so daß der Mantel selbständig zerfällt. Die Gasentwicklung verhindert, daß sich an den Grenzflächen zwischen Feststoff und Wasser stabile Lösungsgleichgewichte ausbilden. Die entweichenden Gasbläschen verwirbeln gesättigte und ungesättigte Lösungen, so daß das Auflösen der Substanzen ingesamt rascher erfolgt. Die Blasenbildung bewirkt ferner ein mechanisches Zerkleinern und Aufbrechen des Mantels.

Der Mantel besteht also aus einem in Wasser schnell sich lösenden und dispergierenden Trägermaterial. Geeignete Trägermaterialien für den Mantel sind: Weinsäure, Zitronensäure und andere physiologisch verträgliche Säuren, wie Äpfelsäure, etc. Weitere Bestandteile sind Alkali- und Erdalkalicarbonate, insbesondere Calciumcarbonat, Natriumhydrogencarbonat, Natriumcarbonat, Kaliumcarbonat, Kaliumhydrogencarbonat, weiters inerte Trägerstoffe wie Sorbitol, Lactose, Mannitol, aber auch Gleitmittel wie Polyäthylenglycol, etc. Der Mantel kann ferner übliche, in Wasser lösliche und dispergierbare Füll- und Trägerstoffe enthalten, wie Zucker, insbesondere Lactose, Fructose und Saccharose, Zuckeralkohole, wie Mannit, Polyvinylpyrrolidon, Mischpolymerisate wie Vinylpyrrolidon/Vinylacetat, Calciumphosphate und/oder Talkum, Cellulose, Ascorbinsäure, lösliche Geschmacksstoffe, Aspartam, etc.

Bevorzugt ist weiter, wenn das Mantelmaterial einen schnell löslichen Wirkstoff enthält. Besonders bevorzugt ist, wenn der Mantel eine Initialdosis und der Kern eine Erhaltungsdosis der Wirksubstanz enthalten. Die im Mantel befindliche Wirksubstanz wird dann beim Lösen des Mantels mitgelöst und ist nach dem Trinken somit für den Körper sofort verfügbar.

Wirkstoffe bzw. Tablettensubstanzen, die sich für eine solche Applikation besonders eignen, sind Acetylcystein, sowie Monofluorophosphat mit Calcium.

Der Tablettenkern kann mit einem auch in Magensaft unlöslichen Film umhüllt sein, so daß die Wirksubstanzen erst im Gastrointestinaltrakt freigesetzt werden. Der Kern kann auch einen quellfähigen Überzug aufweisen und die Wirksubstanzen im Magen freisetzen. Eine quellfähige Hülle des Tablettenkerns fördert im übrigen auch den Zerfall des Mantels.

Ein weiterer Vorteil einer derartigen pharmazeutischen Formulierung liegt darin, daß Mantel und Kern verschiedene Wirkstoffe enthalten können. Da die Wirkstoffe bei einer entsprechenden Umhüllung des Kerns auch nach der Einnahme fast vollständig voneinander getrennt sind, können so selbst unverträgliche Wirkstoffe in einer Formulierung verarbeitet werden. Beispiele hiefür sind Ergotamintartrat und Acetylsalicylsäure. Beispiele für eine Initialdosis im Mantel und eine Erhaltungsdosis im Retardkern sind Acetylcystein und Natriumfluorophosphat. Die Mantelschicht enthält bevorzugt reinen Wirkstoff, der dann beim Lösen des Mantels mit in Lösung geht. Die Wirkstoffe werden hierbei nach dem Trinken sofort resorbiert und erreichen rasch einen therapeutischen Bereich im Blut. Der Wirkstoff im Kern hingegen ist retardiert, und seine Bioverfügbarkeit kann in bekannter Weise auf einen Zeitraum von 2 bis 18 Stunden eingestellt werden.

Besonders geeignet als Tablettenkerne sind sognannte Matrixtabletten mit einem Schwammgerüst, bestehend aus Polyacrylaten oder Cellulosederivaten, die ihrerseits wieder guellfähig sind. Ebenso sind Fettmatrizes, z.B. aus Lipidrol, bestens geeignet. Bevorzugt wird die Kombination einer retardierten Wirkstoffmatrix mit einem sie umgebenden Retardfilm. In diesem Fall ergibt sich bezüglich der Retardierung ein überadditiver Effekt.

Der Kern kann selbstverständlich pharmazeutische Hilfsstoffe und Bindemittel enthalten. Bevorzugte Bindemittel sind Stärkekleister, Gummi arabicum, Gelatine, Cellulosealkylderivate, Cellulose-ätherderivate und Celluloseesterderivate.

Selbstverständlich können Mantel und/oder Kern zusätzlich Süßstoffe, Aromastoffe, Geschmacksverstärker und -verbesserer sowie künstliche und natürliche Farbstoffe enthalten. Der Kern kann ferner Gleit- und Schmiermittel, wie hochdisperse Kieselsäure, Magnesiumstearat, etc. aufweisen. Enthält der Kern wirkstoffhaltige Pellets oder Partikel, so ist ferner die Anwesenheit von Suspendiermitteln, wie wasserlösliche Celluloseäther, Carboxymethylcellulosen, Pektine und Alginate vorteilhaft.

Weitere Ziele, Merkmale und Vorteile der Erfindung ergeben sich aus der nachstehenden Beschreibung und den Ausführungsbeispielen in Verbindung mit den Zeichnungen. Es zeigen:
- Fig.1: einen Schnitt durch eine erfindungsgemäße Mantellöstablette und, schematisch, deren Applikation;
- Fig.2: einen Schnitt durch eine Mantellöstablette mit umhülltem Kern und Wirkstoffpellets;
- Fig.3: das Freisetzungsprofil einer erfindungsgemäßen Manteltablette.

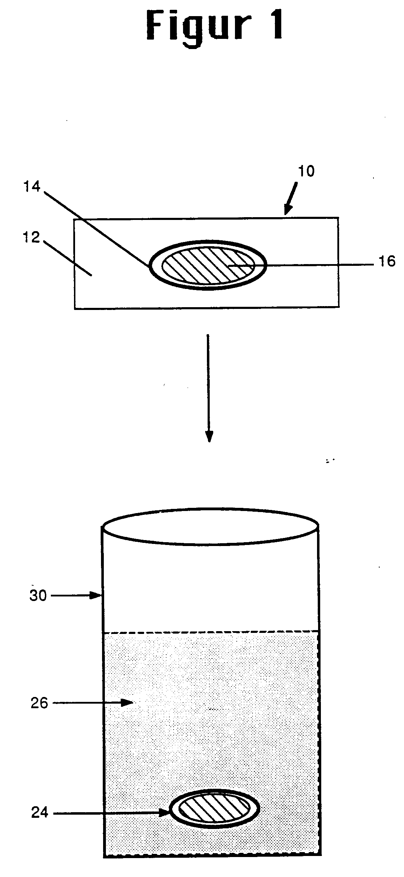

Fig. 1 zeigt eine Manteltablette 10 und schematisch deren Applikation. Der zwischen 5 und 8 mm im Durchmesser große Tablettenkern ist von einem in Wasser quellfähigen Film 14 umgeben. Auch die Retardmatrix ist quellfähig, so daß der Brausemantel 12 beim Lösen der Tablette in Wasser gesprengt wird und so rascher in Lösung geht.

Zur Einnahme wird die Brausemanteltablette 10 in ein Glas 30 mit Wasser gegeben, wo sich der Mantel bei Raumtemperatur selbstständig innerhalb 30 bis 180 Sekunden löst. Der Brausemantel 12 enthält neben den Brausemitteln auch nichtummantelten Wirkstoff in Pulver-, Kristall- oder Mikrokristallform, so daß nach dem Trinken des Brausegetränks schnell eine therapeutische Wirkung eintritt. Der Brausemantel enthält ferner Aroma- und Geschmackstoffe, so daß nach Lösen des Mantels ein gefälliges, aromatisches Brausegetränk 26 vorliegt - am Boden des Getränks befindet sich dann der Tablettenkern 24.

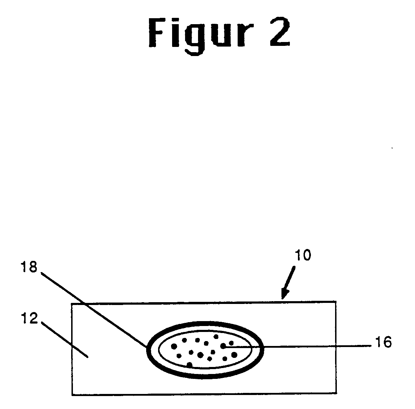

Fig. 2 zeigt eine Manteltablette 10, wo der Wirkstoff 16 im Tablettenkern in Pellets vorliegt. Die Pellets sind von einem retardierenden, quellfähigen Film 18 umgeben, so daß die Wirkstofffreisetzung in mehrfacher Hinsicht retardiert ist.

### Beispiel 1

### (Manteltablette mit Calcium und MFP als Wirkstoff)

| | | |
|---|---|---|
| Mantelhülle: (nicht retardiert) | Calciumcarbonat | 2,50 g |
| | Zitronensäure | 4,30 g |
| | Natriumcyclamat | 40,00 mg |
| | Saccharin-Natrium | 6,00 mg |
| | Aroma | 40,00 mg |
| Tablettenkern: | | |
| Retardtablette mit | Dinatriummonofluorophosphat (Retard-Wirkstoff) | 152,00 mg |
| | Eudragit RSPM | 63,55 mg |
| | Eudragit RS 30 D | 9,36 mg |
| | Wax E | 3,00 mg |
| | Boeson VP | 3,00 mg |
| | Stearinsäure | 4,00 mg |
| | Aerosil 200 | 1,00 mg |
| | Talk | 2,90 mg |
| | Titandioxid | 2,05 mg |
| | Diäthylphthalat | 0,34 mg |
| | Natriumalginat | 26,20 mg |
| Lösungszeit des Mantels in 100 ml Wasser: 130 Sekunden. | | |

### Beispiel 2

### (Manteltablette mit Calcium und MFP als nichtretardiertem Wirkstoff, sowie zusätzlichem MFP als Erhaltungsdosis)

| | | |
|---|---|---|
| Hülle: | Calciumcarbonat | 2,50 g |
| | Zitronensäure | 4,30 g |
| | Aspartam | 40,00 mg |
| | Aroma | 40,00 mg |
| | Natriummonofluorphosphat | 52,00 mg |
| Kern: | | |
| Retardtablette mit | Natriummonofluorphosphat | 100,00 mg |
| | Eudragit RSPM | 40,00 mg |
| | Eudragit RS 30 D | 6,00 mg |
| | Stearinsäure | 4,00 mg |
| | Talk | 5,00 mg |
| | Titandioxid | 2,00 mg |
| | Diäthylenglycol | 1,00 mg |
| | Hydoxypropylmethylcellulose | 26,20 mg |
| Lösungszeit des Mantels in 100 ml Wasser: 115 Sekunden. | | |

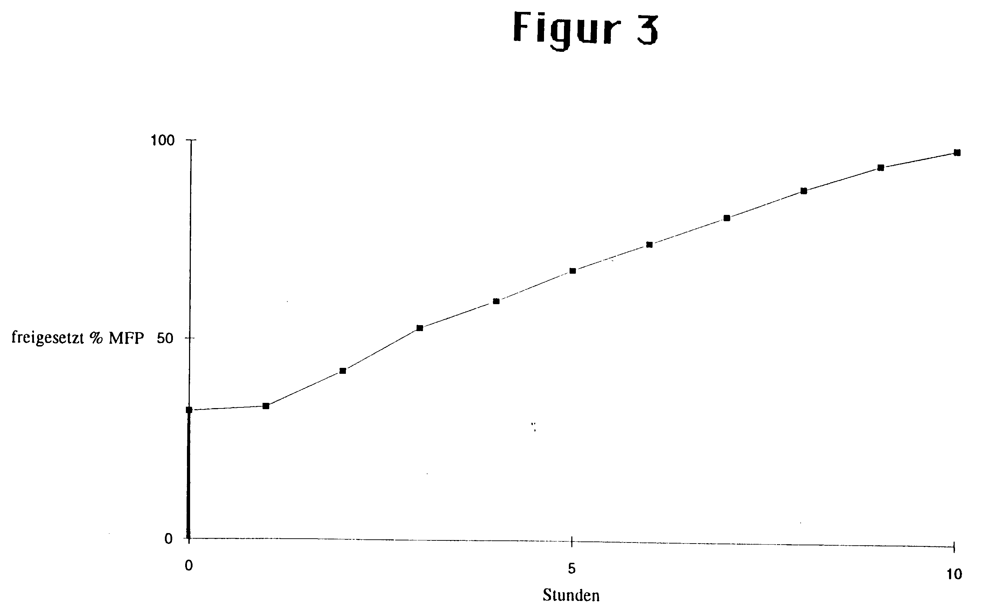

Fig. 3 zeigt das Profil der Freisetzung bei einer Tablette der obigen Zusammensetzung. Das Freisetzungsprofil wurde nach der Paddle-Methode bestimmt. Die Tablettenaufnahme erfolgte, wie schematisch in Fig. 1 dargestellt, durch Lösen der Tablette in 100 ml Wasser und anschließendem Trinken des Brausegetränks einschließlich des Tablettenkerns.

## Patentansprüche (Patentansprüche für folgende(n) Vertragsstaat(en): AT, BE, CH, DE, FR, GB, IT, LI, NL)

1. Pharmazeutische Formulierung zur oralen Applikation, bestehend aus einer einen Kern bildenden und wenigstens einen Wirkstoff enthaltenden Retardtablette, die völlig von einem Mantel umgeben ist, der wenigstens einen Wirkstoff und Brausematerial enthält und sich in 100 ml Wasser zwischen 30 und 180 Sekunden, vorzugsweise zwischen 90 und 120 Sekunden, löst oder dispergiert.

2. Pharmazeutische Formulierung nach Anspruch 1, dadurch gekennzeichnet, daß der Tablettenkern zur Beschleunigung des Zerfalls quellfähig ist und/oder eine quellfähige Hülle aufweist.

3. Pharmazeutische Formulierung nach Anspruch 1 oder 2, dadurch gekennzeichnet, daß der Mantel eine Initialdosis und der Kern eine Erhaltungsdosis von einem oder mehreren Wirkstoffen enthalten.

4. Pharmazeutische Formulierung nach einem der Ansprüche 1 bis 3, dadurch gekennzeichnet, daß Mantel und Kern inkompatible Wirkstoffe enthalten.

5. Pharmazeutische Formulierung nach einem der Ansprüche 1 bis 4, dadurch gekennzeichnet, daß der Kern suspendierbare Pellets mit Wirksubstanz aufweist.

6. Pharmazeutische Formulierung nach einem der Ansprüche 1 bis 5, dadurch gekennzeichnet, daß der Mantel Süßstoffe, Aromastoffe, Farbstoffe und/oder Geschmacksverstärker aufweist.

7. Pharmazeutische Formulierung nach einem der Ansprüche 1 bis 6, dadurch gekennzeichnet, daß der Mantel über dem Tablettenkern zwischen 1 und 15 g, bevorzugt zwischen 2 und 10 g, wiegt.

8. Verwendung einer pharmazeutischen Formulierung gemäß einem der Ansprüche 1 bis 7 zur Herstellung einer Lösung oder Dispersion der den Mantel der Tablette bildenden Substanzen unter Aufrechterhaltung des im wesentlichen ungelösten Tablettenkerns.

## Patentansprüche (Patentansprüche für folgende(n) Vertragsstaat(en): ES)

1. Verfahren zur Herstellung einer pharmazeutischen Formulierung zur oralen Applikation, bei dem eine einen Kern bildende und wenigstens einen Wirkstoff enthaltende Retardtablette völlig von einem Mantel umhüllt wird, der wenigstens einen Wirkstoff und Brausematerial enthält und sich in 100 ml Wasser zwischen 30 und 180 Sekunden, vorzugsweise zwischen 90 und 120 Sekunden, löst oder dispergiert.

2. Verfahren nach Anspruch 1, dadurch gekennzeichnet, daß der Tablettenkern zur Beschleunigung des Zerfalls mit quellfähigen Bestandteilen und/oder einer quellfähigen Hülle versehen wird.

3. Verfahren nach Anspruch 1 oder 2, dadurch gekennzeichnet, daß den Materialien für den Mantel eine Initialdosis und dem Kern eine Erhaltungsdosis von einem oder mehreren Wirkstoffen zugesetzt werden.

4. Verfahren nach einem der Ansprüche 1 bis 3, dadurch gekennzeichnet, daß den Materialien für den Mantel einerseits und den Materialien für den Kern andererseits miteinander inkompatible Wirkstoffe zugesetzt werden.

5. Verfahren nach einem der Ansprüche 1 bis 4, dadurch gekennzeichnet daß den Materialien für den Kern die Wirksubstanz in Form suspendierbarer Pellets zugesetzt wird.

6. Verfahren nach einem der Ansprüche 1 bis 5, dadurch gekennzeichnet, daß den Materialien für den Mantel Süßstoffe, Aromastoffe, Farbstoffe und/oder Geschmacksverstärker zugesetzt werden.

7. Verfahren nach einem der Ansprüche 1 bis 6, dadurch gekennzeichnet, daß der Mantel über dem Tablettenkern auf ein Gewicht zwischen 1 und 15 g, bevorzugt zwischen 2 und 10 g, eingestellt wird.

8. Pharmazeutische Formulierung zur oralen Applikation, bestehend aus einer einen Kern bildenden und wenigstens einen Wirkstoff enthaltenden Retardtablette, die völlig von einem Mantel umgeben ist, der wenigstens einen Wirkstoff und Brausematerial enthält und sich in 100 ml Wasser zwischen 30 und 180 Sekunden, vorzugsweise zwischen 90 und 120 Sekunden, löst oder dispergiert.

9. Pharmazeutische Formulierung nach Anspruch 8, dadurch gekennzeichnet, daß der Tablettenkern zur Beschleunigung des Zerfalls quellfähig ist und/oder eine quellfähige Hülle aufweist.

10. Pharmazeutische Formulierung nach Anspruch 8 oder 9, dadurch gekennzeichnet, daß der Mantel eine Initialdosis und der Kern eine Erhaltungsdosis von einem oder mehreren Wirkstoffen enthalten.

11. Pharmazeutische Formulierung nach einem der Ansprüche 8 bis 10, dadurch gekennzeichnet, daß Mantel und Kern inkompatible Wirkstoffe enthalten.

12. Pharmazeutische Formulierung nach einem der Ansprüche 8 bis 11, dadurch gekennzeichnet, daß der Kern suspendierbare Pellets mit Wirksubstanz aufweist.

13. Pharmazeutische Formulierung nach einem der Ansprüche 8 bis 12, dadurch gekennzeichnet, daß der Mantel Süßstoffe, Aromastoffe, Farbstoffe und/oder Geschmacksverstärker aufweist.

14. Pharmazeutische Formulierung nach einem der Ansprüche 8 bis 13, dadurch gekennzeichnet, daß der Mantel über dem Tablettenkern zwischen 1 und 15 g, bevorzugt zwischen 2 und 10 g, wiegt.

15. Verwendung einer pharmazeutischen Formulierung gemäß einem der Ansprüche 8 bis 14 zur Herstellung einer Lösung oder Dispersion der den Mantel der Tablette bildenden Substanzen unter Aufrechterhaltung des im wesentlichen ungelösten Tablettenkerns.

## Claims (Claims for the following Contracting State(s): AT, BE, CH, DE, FR, GB, IT, LI, NL)

1. Pharmaceutical formulation for oral administration, consisting of a sustained-release tablet forming a core, containing at least one active ingredient and completely surrounded by a press coat which contains at least one active ingredient and effervescent material and dissolves or disperses in 100 ml of water in from 30 to 180 seconds, preferably from 90 to 120 seconds.

2. Pharmaceutical formulation according to Claim 1, characterized in that the tablet core is swellable and/or has a swellable coat for accelerating the disintegration.

3. Pharmaceutical formulation according to Claim 1 or 2, characterized in that the press coat contains an initial dose, and the core a maintenance dose, of one or more active ingredients.

4. Pharmaceutical formulation according to any of Claims 1 to 3, characterized in that press coat and core contain incompatible active ingredients.

5. Pharmaceutical formulation according to any of Claims 1 to 4, characterized in that the core comprises suspendable pellets containing active ingredient.

6. Pharmaceutical formulation according to any of Claims 1 to 5, characterized in that the press coat contains sweeteners, flavours, colours and/or flavour enhancers.

7. Pharmaceutical formulation according to any of Claims 1 to 6, characterized in that the press coat over the tablet core weighs between 1 and 15 g, preferably between 2 and 10 g.

8. Use of a pharmaceutical formulation according to any of Claims 1 to 7 for the preparation of a solution or dispersion of the substances forming a press coat of the tablet, while maintaining the essentially undissolved tablet core.

## Claims (Claims for the following Contracting State(s): ES)

1. Process for the preparation of a pharmaceutical formulation for oral administration, in which a sustained-release tablet forming a core and containing at least one active ingredient is surrounded by a press coat which contains at least one active ingredient and effervescent material and dissolves or disperses in 100 ml of water in from 30 to 180 seconds, preferably from 90 to 120 seconds.

2. Process according to Claim 1, characterized in that the tablet core is provided with swellable components and/or a swellable coat for acceleration of the disintegration.

3. Process according to Claim 1 or 2, characterized in that an initial dose of one or more active ingredients is added to the materials for the press coat and a maintenance dose of one or more active ingredients is added to the core.

4. Process according to any of Claims 1 to 3, characterized in that active ingredients incompatible with one another are added to the materials for the press coat on the one hand and to the materials for the core on the other hand.

5. Process according to any of Claims 1 to 4, characterized in that the active ingredient is added in the form of suspendable pellets to the materials for the core.

6. Process according to any of Claims 1 to 5, characterized in that sweeteners, flavours, colours and/or flavour enhancers are added to the materials for the press coat.

7. Process according to any of Claims 1 to 6, characterized in that the press coat over the tablet core is adjusted to a weight between 1 and 15 g, preferably between 2 and 10 g.

8. Pharmaceutical formulation for oral administration, consisting of a sustained-release tablet forming a core, containing at least one active ingredient and completely surrounded by a press coat which contains at least one active ingredient and effervescent material and dissolves or disperses in 100 ml of water in from 30 to 180 seconds, preferably from 90 to 120 seconds.

9. Pharmaceutical formulation according to Claim 8, characterized in that the tablet core is swellable and/or has a swellable coat for accelerating the disintegration.

10. Pharmaceutical formulation according to Claim 8 or 9, characterized in that the press coat contains an initial dose, and the core a maintenance dose, of one or more active ingredients.

11. Pharmaceutical formulation according to any of Claims 8 to 10, characterized in that press coat and core contain incompatible active ingredients.

12. Pharmaceutical formulation according to any of Claims 8 to 11, characterized in that the core comprises suspendable pellets containing active ingredient.

13. Pharmaceutical formulation according to any of Claims 8 to 12, characterized in that the press coat contains sweeteners, flavours, colours and/or flavour enhancers.

14. Pharmaceutical formulation according to any of Claims 8 to 13, characterized in that the press coat over the tablet core weighs between 1 and 15 g, preferably between 2 and 10 g.

15. Use of a pharmaceutical formulation according to any of Claims 8 to 14 for the preparation of a solution or dispersion of the substances forming a press coat of the tablet, while maintaining the essentially undissolved tablet core.

## Revendications (Revendications pour l'(les) Etat(s) contractant(s) suivant(s): AT, BE, CH, DE, FR, GB, IT, LI, NL)

1. Formulation pharmaceutique pour application orale, constituée d'un comprimé à retard constituant un noyau et contenant au moins un principe actif, comprimé entier entouré d'une enveloppe contenant au moins un principe actif et un matériau effervescent et se dissolvant ou se dispersant dans 100 ml d'eau, en une durée comprise entre 30 et 180 secondes, de préférence entre 90 et 120 secondes.

2. Formulation pharmaceutique selon la revendication 1, caractérisée en ce que le noyau de comprimé est susceptible de gonfler et/ou contient une enveloppe gonflable, en vue d'accélérer la désagrégation.

3. Formulation pharmaceutique selon la revendication 1 ou 2, caractérisée en ce que l'enveloppe contient une dose initiale et le noyau contient une dose d'entretien, d'un ou plusieurs principes actifs.

4. Formulation pharmaceutique selon l'une des revendications 1 à 3, caractérisée en ce que l'enveloppe et le noyau contiennent des principes actifs incompatibles.

5. Formulation pharmaceutique selon l'une des revendications 1 à 4, caractérisée en ce que le noyau présente des boulettes susceptibles d'être mises en suspension et comportant une substance active.

6. Formulation pharmaceutique selon l'une des revendications 1 à 5, caractérisée en ce que l'enveloppe présente des édulcorants, des substances aromatiques, des colorants et/ou des amplificateurs de goût.

7. Formulation pharmaceutique selon l'une des revendications 1 à 6, caractérisée en ce que l'enveloppe placée sur le noyau du comprimé pèse entre 1 et 15 g, de préférence entre 2 et 10 g.

8. Utilisation d'une formulation pharmaceutique selon l'une des revendications 1 à 7, pour la préparation d'une solution ou d'une dispersion des substances formant l'enveloppe du comprimé, avec conservation du noyau de comprimé sensiblement non dissous.

## Revendications (Revendications pour l'(les) Etat(s) contractant(s) suivant(s): ES)

1. Procédé de préparation d'une formulation pharmaceutique pour application orale, dans lequel un comprimé à retard formant un noyau et contenant au moins une substance active est entièrement enveloppé par une enveloppe, enveloppe contenant au moins un principe actif et un matériau effervescent et se dissolvant ou se dispersant dans 100 ml d'eau sur une durée comprise entre 30 et 180 secondes, de préférence entre 90 et 120 secondes.

2. Procédé selon la revendication 1, caractérisé en ce que le noyau de comprimé est pourvu de composants susceptibles de gonfler et/ou d'une enveloppe susceptible de gonfler, en vue d'accélérer la désagrégation.

3. Procédé selon la revendication 1 ou 2, caractérisé en ce qu'on ajoute aux matériaux de l'enveloppe une dose initiale et au noyau une dose d'entretien, constituée d'un ou plusieurs principes actifs.

4. Procédé selon l'une des revendications 1 à 3, caractérisé en ce qu'on ajoute aux matériaux de l'enveloppe, d'une part, et aux matériaux du noyau, d'autre part, des principes actifs incompatibles entre eux.

5. Procédé selon l'une des revendications 1 à 4, caractérisé en ce qu'on ajoute aux matériaux du noyau la substance active sous une forme de boulettes susceptibles d'être mises en suspension.

6. Procédé selon l'une des revendications 1 à 5, caractérisé en ce qu'on ajoute aux matériaux destinés à l'enveloppe des édulcorants, des substances aromatiques, des colorants et/ou des amplificateurs de goût.

7. Procédé selon l'une des revendications 1 à 6, caractérisé en ce que l'enveloppe placée au-dessus du noyau de comprimé est réglée à un poids compris entre 1 et 15 g, de préférence entre 2 et 10 g.

8. Formulation pharmaceutique pour application orale, constituée d'un comprimé à retard constituant un noyau et contenant au moins un principe actif, comprimé entièrement entouré d'une enveloppe, qui contient au moins un principe actif et un matériau effervescent et qui se dissout ou se disperse dans 100 ml d'eau, sur une durée comprise entre 30 et 180 secondes, de préférence entre 90 et 120 secondes.

9. Formulation pharmaceutique selon la revendication 8, caractérisée en ce que le noyau de comprimé est susceptible de gonfler et/ou présente une enveloppe susceptible de gonfler, en vue d'accélérer la désagrégation.

10. Formulation pharmaceutique selon la revendication 8 ou 9, caractérisée en ce que l'enveloppe contient une dose initiale et le noyau contient une dose d'entretien, d'un ou plusieurs principes actifs.

11. Formulation pharmaceutique selon l'une des revendications 8 à 10, caractérisée en ce que l'enveloppe et le noyau contiennent des principes actifs incompatibles.

12. Formulation pharmaceutique selon l'une des revendications 8 à 11, caractérisée en ce que le noyau présente des boulettes susceptibles d'être mises en suspension, avec un principe actif.

13. Formulation pharmaceutique selon l'une des revendications 8 à 12, caractérisée en ce que l'enveloppe contient des édulcorants, des substances aromatiques, des colorants et/ou des amplificateurs de goût.

14. Formulation pharmaceutique selon l'une des revendications 8 à 13, caractérisée en ce que l'enveloppe placée au-dessus du noyau de comprimé est d'un poids compris entre 1 et 15 g, de préférence entre 2 et 10 g.

15. Utilisation d'une formulation pharmaceutique selon l'une des revendications 8 à 14 pour la préparation d'une solution ou d'une dispersion des substances constituant l'enveloppe du comprimé, avec conservation du noyau de comprimé pratiquement non dissous.
